Europäisches Patentamt

European Patent Office

Office européen des brevets

(11) Veröffentlichungsnummer: **0 136 732 B1**

## (12) EUROPÄISCHE PATENTSCHRIFT

(45) Veröffentlichungstag der Patentschrift: **25.09.91**

(51) Int. Cl.⁵: **G01T 1/164**

(21) Anmeldenummer: **84201042.3**

(22) Anmeldetag: **12.07.84**

(54) **Röntgengerät.**

(30) Priorität: **27.07.83 DE 3327031**

(43) Veröffentlichungstag der Anmeldung:
**10.04.85 Patentblatt 85/15**

(45) Bekanntmachung des Hinweises auf die
Patenterteilung:
**25.09.91 Patentblatt 91/39**

(84) Benannte Vertragsstaaten:
**DE FR GB**

(56) Entgegenhaltungen:
**WO-A-82/01124**
**US-A- 4 091 416**
**US-A- 4 179 100**

(73) Patentinhaber: **Philips Patentverwaltung GmbH**
**Wendenstrasse 35 Postfach 10 51 49**
**W-2000 Hamburg 1(DE)**

(84) Benannte Vertragsstaaten:
**DE**

Patentinhaber: **N.V. Philips' Gloeilampenfa-brieken**
**Groenewoudseweg 1**
**NL-5621 BA Eindhoven(NL)**

(84) Benannte Vertragsstaaten:
**FR GB**

(72) Erfinder: **Kayser, Harald**
**Heisterkamp 26**
**W-2000 Wedel(DE)**

(74) Vertreter: **Hartmann, Heinrich, Dipl.-Ing. et al**
**Philips Patentverwaltung GmbH Wenden-strasse 35 Postfach 10 51 49**
**W-2000 Hamburg 1(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Röntgengerät mit einem Röntgenstrahler und einer Blendenanordnung zum Erzeugen eines fächerförmigen, einen Untersuchungsbereich durchsetzenden Strahlenbündels, mit einer flächigen Szintillatoranordnung zum Umsetzen des durch das Strahlenbündel vom Untersuchungsbereich erzeugten zeilenförmigen Röntgenschattenbildes in ein längerwelliges Strahlungsbild, einer mit der Szintillatoranordnung gekoppelten Lichtleiteranordnung, einer Wandleranordnung, die das Bild sequentiell in elektrische Signale umsetzt und mit einem Speicher zum Speichern der elektrischen Signale.

Ein solches Röntgengerät ist aus der US-PS 4,179,100 bekannt.

Ein Nachteil des bekannten Röntgengerätes besteht darin, daß die Intensität des dem Wandler zugeführten Lichtes sehr gering ist, was entweder die Verwendung von hochempfindlichen Wandlerelementen bedingt oder aber zur Folge hat, daß dem Patienten bei einer Untersuchung eine verhältnismäßig hohe Dosis verabfolgt wird.

Dieser Nachteil wird bei dem Röntgengerät nach der PTC-Veröffentlichung WO 82/01124 dadurch vermieden, daß das zeilenförmige Röntgenschattenbild auf den Eingangsschirm eines Röntgenbildverstärkers trifft, auf dessen Ausgangsschirm somit ein sichtbares Bild des Röntgenstrahlenreliefs erscheint. Ein Nachteil des bekannten Röntgengerätes besteht darin, daß der Bildverstärker im Strahlengang angeordnet werden muß und verhältnismäßig voluminös ist, obwohl eigentlich nur ein Bruchteil der Fläche seines Eingangsschirmes zur Bildumsetzung benötigt wird.

Ein weiterer Nachteil des aus der US-PS 4,179,100 bekannten Röntgengerätes besteht darin, daß die Lichtleiteranordnungen (Fig. 8) geordnet sein müssen, so daß die von der Wandleranordnung erzeugten zeitlich aufeinanderfolgenden Abtastwerte dem räumlichen Verlauf des Röntgenschattenbildes entsprechen. Derartig geordnete Lichtleiterbündel sind aber relativ teuer.

Aufgabe der vorliegenden Erfindung ist es, ein Röntgengerät der eingangs genannten Art so auszugestalten, daß sich am Eingang der Wandleranordnung ein insgesamt helleres Bild ergibt und daß auch ungeordnete Lichtleiteranordnungen verwendet werden können.

Erfindungsgemäß wird diese Aufgabe in einem gattungsgemäßen Röntgengerät dadurch gelöst, daß die Szintillatoranordnung über die Lichtleiteranordnung mit einem Bildverstärker gekoppelt ist, dessen Ausgangsbild auf die Wandleranordnung abgebildet wird, und daß eine Sortierschaltung vorgesehen ist, die die Abtastwerte des elektrischen Signals beim Einschreiben in bzw. beim Auslesen aus dem Speicher so vertauscht, daß ihre Folge der Folge der Helligkeitswerte in dem Röntgenschattenbild entspricht.

Die Helligkeit wird bei der Erfindung also durch die Verwendung eines Bildverstärkers vergrößert. Im Gegensatz zu den in der Röntgentechnik im allgemeinen verwendeten Röntgenbildverstärkern wird der Eingangsschirm dieses Bildverstärkers nicht mit Röntgenstrahlung beaufschlagt, sondern durch das über die Lichtleiteranordnung übertragene Licht. Der Bildverstärker muß daher nicht im Strahlengang angeordnet sein, sondern kann an geeigneter Stelle im Gerät angeordnet werden. Der Bildverstärker hat dabei die Funktion eines Restlichtverstärkers, wie sie z.B. in Nachtsichtgeräten verwendet werden, und kann mit sogenannten Vielkanalplatten ausgerüstet sein. Derartige Bildverstärker haben - verglichen mit Röntgenbildverstärkern - ein relativ kleines Volumen; daß die Eingangsfläche, deren Bild in ein helleres Ausgangsbild umgesetzt wird, dabei wesentlich kleiner ist als bei Röntgenbildverstärkern, stört nicht, weil auch die Fläche, die das zeilenförmige Röntgenschattenbild einnimmt, relativ klein ist.

Das Ausgangsbild des Bildverstärkers wird durch die Wandleranordnung sequentiell in ein elektrisches Signal umgesetzt, das als Folge von Abtastwerten in einem Speicher gespeichert wird. Da die Lichtleiteranordnung nicht geordnet ist, werden in der Regel die in adressenmäßig benachbarten Speicherplätzen gespeicherten Abtastwerte nicht den Helligkeitswerten in benachbarten Punkten des zeilenförmigen Röntgenschattenbildes entsprechen. Um diese Kongruenz zu erreichen, ist die Sortierschaltung vorgesehen. Diese Sortierschaltung basiert auf folgender Überlegung: Jedem Punkt in dem zeilenförmigen Röntgenschattenbild bzw. jedem Punkt am Eingang der Lichtleiteranordnung entspricht irgendein Punkt am Ausgang der Lichtleiteranordnung bzw. am Ausgang des Bildverstärkers. Dieser letztgenannte Punkt wird durch die Wandleranordnung in ein elektrisches Signal umgesetzt und - gegebenenfalls über einen Analog-Digital-Wandler - in den Speicher eingeschrieben, so daß letztendlich jedem Punkt in dem zeilenförmigen Röntgenschattenbild ein bestimmter Zeitpunkt in dem von der Wandleranordnung gelieferten elektrischen Signal bzw. ein bestimmter Platz in der zu speichernden bzw. gespeicherten Folge von Abtastwerten entspricht. Die Sortierschaltung sortiert die Abtastwerte nun so, daß beim Auslesen des Speichers die Folge der Abtastwerte der Folge der ihnen zugeordneten Bildpunkte in dem zeilenförmigen Röntgenschattenbild entspricht.

Die Erfindung wird nachstehend anhand der Zeichnung näher erläutert. Es zeigen

    Fig. 1    ein Teil eines Ausführungsbeispiels der Erfindung,

Fig. 2 bei der Erfindung auftretende Signalverläufe und

Fig. 3 ein erstes Ausführungsbeispiel einer Sortierschaltung,

Fig. 4 eine Schaltung zum Programmieren des bei der Sortierschaltung erforderlichen programmierbaren Festwertspeichers und

Fig. 5 eine andere Ausführungsform der Sortierschaltung.

Die von einem Röntgenstrahler 1 ausgehende Röntgenstrahlung 2 wird durch Schlitzblenden 3, die zwischen dem Röntgenstrahler und dem Untersuchungsbereich angeordnet sind, zu einem fächerförmigen Röntgenstrahlenbündel 4 ausgeblendet, das den im Untersuchungsbereich angeordneten Patienten 5 durchsetzt und auf eine flächige Szintillatoranordnung, beispielsweise eine lichtemittierende Verstärkerfolie 6 trifft, und auf dieser ein zeilenförmiges Bild erzeugt, d.h. ein Bild, das in Zeilenrichtung z.B. 300 mm mißt und senkrecht dazu wesentlich geringere Abmessungen hat, z.B. 0,15 mm. Dieses Bild wird auf noch zu beschreibende Weise einem Speicher zugeführt, was die Verarbeitung durch einen Computer vor der Wiedergabe auf einem Wiedergabegerät ermöglicht.

Nachdem die in den verschiedenen Punkten des zeilenförmigen Röntgenschattenbildes auftretenden Intensitätswerte der Röntgenstrahlung gemessen und gespeichert sind, erfolgt eine Relativbewegung zwischen dem Patienten 5 und dem Röntgenstrahlenbüdel - beispielsweise durch Verschiebung der den Patienten tragenden nicht näher dargestellten Tischplatte senkrecht zum Röntgenstrahlenbündel - , so daß ein dem zuvor durchstrahlten Körperquerschnitt benachbarter Körperquerschnitt auf der Szintillatoranordnung 6 abgebildet wird. Dies wiederholt sich so lange, bis am Ende ein Gesamtbild gespeichert ist, dessen Abmessungen in der einen Richtung der Länge einer Zeile und in der dazu senkrechten Richtung der Breite einer Zeile multipliziert mit der Zahl der Verschiebungsschritte entsprechen. Dabei kann gegebenenfalls noch zwischen dem Untersuchungsbereich bzw. dem Patienten und der Szintillatoranordnung 6 eine weitere Schlitzblende angeordnet sein, die die - schon stark verringerte - Streustrahlung aus dem Objekt noch weiter reduziert.

In innigem Kontakt mit der szintillierenden Oberfläche der Szintillatoranordnung steht ein Lichtleitfaserbündel 8, so daß das durch die Röntgenquanten erzeugte sichtbare Licht nahezu verlustlos in das Lichtleitfaserbündel eintritt. Die Stirnflächen der Lichtleitfasern sind dabei in Zeilenrichtung nebeneinander angeordnet und erfassen somit gerade das durch die Schlitzblenden ausgeblendete Röntgenstrahlenbündel. Gegebenenfalls kann auch unmittelbar auf die Stirnflächen der Lichtleitfaser eine die Röntgenstrahlung in sichtbares Licht umsetzende Szintillatorschicht abgeschieden sein.

Das von der Szintillatoranordnung 6 abgewandte Ende des Lichtleitfaserbündels 8 ist beliebig zusammengeführt, so daß sich eine Austrittsfläche 9 mit z.B. kreisrundem Querschnitt ergibt. Diese polierte Austrittsfläche ist an den Eingang eines flachen Bildverstärkers 10 angekoppelt, der aufgrund seines inneren Aufbaues auf der Ausgangsseite ein exaktes optisches Abbild der Eingangsseite liefert, jedoch mit wesentlich größerer Leuchtdichte. Derartige Bildverstärker sind bekannt. Sie werden als Restlichtverstärker z.B. von Nachtsichtgeräten eingesetzt und können u.a. sogenannte Vielkanal-Platten enthalten.

Der Ausgang des Bildverstärkers 10 wird auf die Eingangsfläche einer Wandleranordnung 12 abgebildet. Dies kann entweder dadurch erfolgen, daß die Ausgangsfläche des Bildverstärkers 10 und die Eingangsfläche der Wandleranordnung 12 unmittelbar miteinander in Kontakt stehen, oder durch eine Linsenanordnung, oder - wie im Ausführungsbeispiel - durch eine zwischengeschaltete Faserplatte 11. Die Wandleranordnung ist ein Halbleiter-Bildaufnehmer, der eine Vielzahl von photoempfindlichen Elementen enthält, die die Helligkeitsverteilung auf einer i.a. rechteckigen Eingangsfläche erfassen und in ein elektrisches Signal am Ausgang 19 umsetzen, wobei die einzelnen Punkte der Eingangsfläche nacheinander zeilenförmig abgetastet werden. Die Abtastgeschwindigkeit wird dabei durch die Frequenz des an der Klemme 20 anliegenden Taktsignals bestimmt. Die Fläche eines photoempfindlichen Elementes ist dabei wesentlich kleiner als der Querschnitt einer Lichtleitfaser, so daß die über eine einzige Lichtleitfaser des Bündels 8 übertragene Information von mehreren photoempfindlichen Elementen erfaßt wird. Derartige Wandleranordnungen sind bekannt - vgl. VALVO-Datenblatt "RGS-Bildaufnehmer" sowie "Funktechnik" 38 (1938) Heft 3, Seiten 110 bis 112. - Grundsätzlich kann als Wandleranordnung jedoch auch eine übliche Fernsehaufnahmeröhre eingesetzt werden.

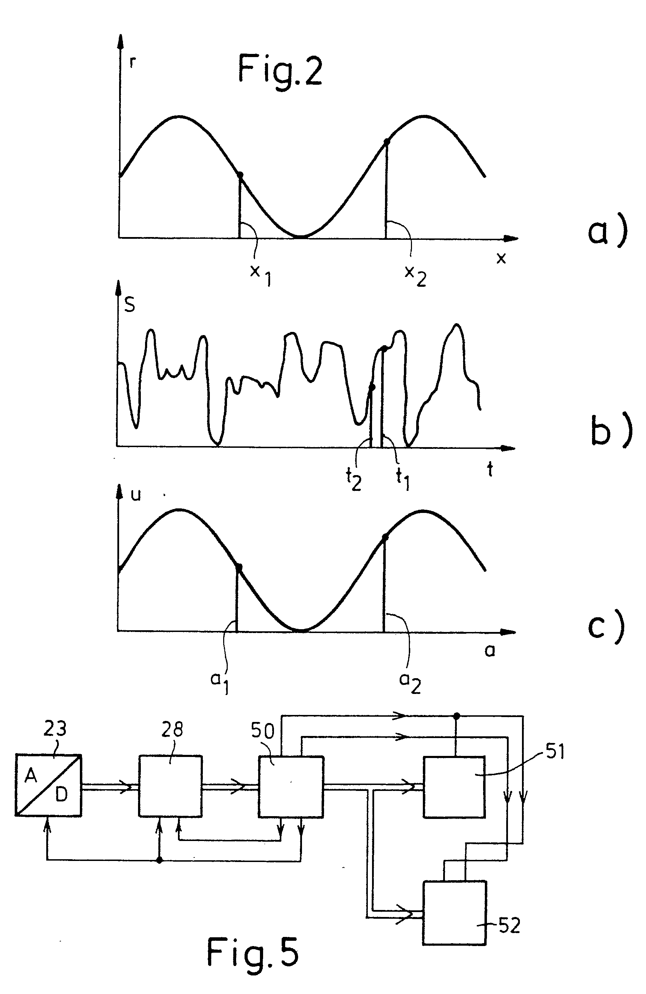

Da die Ausgangsfläche 9 des Lichtleitfaserbündels 8 zu einer kreisrunden (oder rechteckigen) Fläche zusammengeführt ist, während die Eingangsfläche dieses Bündels auf einer Geraden liegt, und da die Abtastung des vom Bildverstärker gelieferten Ausgangsbildes durch die Wandleranordnung unabhängig von der Lage der einzelnen Fasern innerhalb des Lichtleitfaserbündels erfolgt, leuchtet es ein, daß der zeitliche Verlauf des Ausgangssignals in aller Regel nicht dem räumlichen Verlauf der Intensität in Zeilenrichtung des Röntgenschattenbildes entsprechen kann. Zur Erläuterung sei auf Fig. 2a und 2b verwiesen, wo in Fig. 2a der räumliche Verlauf der Intensität r als Funktion des Ortes x (in Zeilenrichtung) dargestellt ist, während b den Verlauf des Ausgangssignales s der Wandleranordnung als Funktion der Zeit darstellt, wobei angenommen ist, daß sich die Strahlungsintensität räumlich sinusförmig über das Röntgenschattenbild verteilt. Das Signal s am Ausgang 19 der Wandleranordnung 12 zeigt als Funktion der Zeit t einen völlig regellosen Verlauf. Ein erkennbarer Zusammenhang zwischen den Verläufen von r und s besteht lediglich insofern als die Extremwerte des Signales s durch die Extremwerte der Intensität r bestimmt werden.

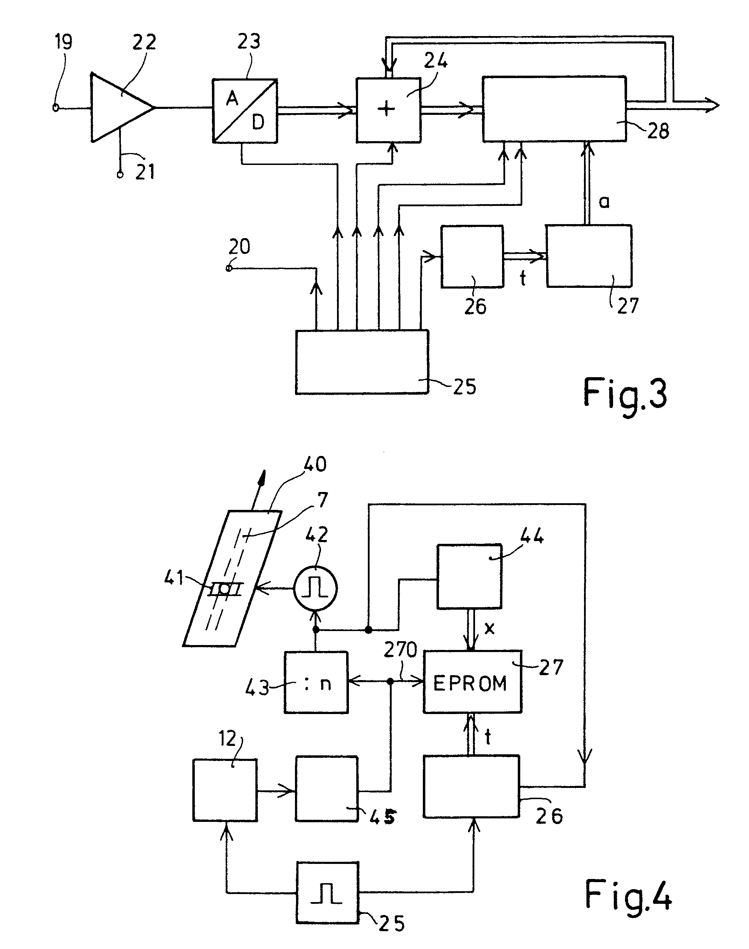

Trotzdem besteht zwischen beiden Signalen ein fester Zusammenhang, der durch die (Un-)Ordnung des Lichtleitfaserbündels bedingt ist. Es sei angenommen, daß die Lichtleitfaser innerhalb des Lichtleitfaserbündels, die am Ort $x_1$ die Intensität überträgt, mit ihrem Ausgang so angeordnet ist, daß diese Intensität bzw. die daraus resultierende Helligkeit zum Zeitpunkt $t_1$ von der Wandleranordnung in ein elektrisches Signal umgesetzt wird, und daß die Lichtleitfaser, die die Intensität r am Ort $x_2$ erfaßt, so angeordnet ist, daß das ihr zugeordnete elektrische Signal zur Zeit $t_2$ erscheint, wobei $t_2$ vor $t_1$ liegt - grundsätzlich aber auch dahinter liegen könnte. Der Abstand zwischen $t_2$ und $t_1$ bezogen auf die Gesamtdauer des Signales ist kleiner als der Abstand zwischen $x_1$ und $x_2$ bezogen auf die Gesamtlänge des Eingangs der Lichtleiteranordnung; jedoch könnte der zeitliche Abstand relativ genau so groß - oder noch größer - sein wie der räumliche Abstand der Lichtleiteranordnung. Somit ist also jedem Punkt (z.B. $x_1$) in dem zeilenförmigen Röntgenschattenbild ein bestimmter Zeitpunkt ($t_1$) in dem Ausgangssignal der Wandleranordnung fest zugeordnet. Diese Tatsache kann dazu benutzt werden, die Signalwerte wieder so zu ordnen, daß ihre Folge der Folge der Intensitätswerte des Röntgenschattenbildes entspricht. Diesem Zweck dient die Sortierschaltung, wozu auf Fig. 3 Bezug genommen wird. Danach wird das Signal vom Ausgang 19 der Wandleranordnung über einen Verstärker 22, dessen Verstärkung durch eine Steuerspannung am Steuereingang 21 einstellbar ist, dem Eingang eines Analog-Digital-Wandlers 23 zugeführt, der in vorgegebenen zeitlichen Abständen, vorzugsweise im Takte des Abtastsignals an der Klemme 20, den Momentanwert des an seinem Eingang liegenden Signales in einen digitalen Abtastwert umsetzt und einem Eingang einer Addierschaltung 24 zuführt, in der es zu einem zweiten Signal hinzuaddiert wird.

Die Taktsignale für den Analog-Digital-Wandler 23 werden von einem Taktsignalgenerator 25 erzeugt, der verschiedene Taktsignale jeweils mit der gleichen Taktfrequenz, jedoch unterschiedlicher Phasenlage und gegebenenfalls auch unterschiedlicher Dauer (kleiner als die Periodendauer) und Amplitude erzeugt. Eines dieser Taktsignale vom Taktsignalgenerator wird einem Zähler 26 zugeführt, der somit an seinem Ausgang ein digitales Signal liefert, das der Zahl der vom Analog-Digital-Wandler seit Beginn der Abtastung des auf der Eingangsfläche der Wandleranordnung erzeugten Bildes entspricht und damit auch einem bestimmten Zeitpunkt (z.B. $t_1$) fest zugeordnet ist. Dieser Ausgang des Zählers 26 ist mit dem Adresseneingang eines programmierbaren Festwertspeichers 27 verbunden, so daß an dessen Ausgang das in dem adressierten Speicherplatz enthaltene Datenwort erscheint. Der Ausgang des programmierbaren Festwertspeichers 27 ist mit dem Adreßeingang eines Schreib-Lese-Speichers 28 verbunden, so daß jedem Abtastwert und somit jedem Zeitpunkt eine bestimmte Adresse zugeordnet ist; so ist z.B. dem zum Zeitpunkt $t_1$ ermittelten Abtastwert die Adresse $a_1$, dem $t_2$ entsprechenden Abtastwert die Adresse $a_2$ zugeordnet usw. Der Speicher 28 besitzt zumindest soviel Speicherplätze wie die Lichtleiteranordnung Lichtleitfasern hat, so daß für jede Lichtleitfaser mindestens ein Speicherplatz mit einer ihr eindeutig zugeordneten Adresse vorgesehen ist. Der Inhalt des so adressierten Speicherplatzes wird unter der Steuerung des Taktsignalgenerators 25 ausgelesen und dem zweiten Eingang der Addierschaltung 24 zugeführt und zu dem jeweiligen Abtastwert des Digital-Analog-Wandlers 23 hinzuaddiert. Das Ergebnis dieser Addition wird - gesteuert durch ein vom Taktsignalgenerator 25 dem Schreibeingang des Speichers 28 zugeführtes Taktsignal - in den zuvor adressierten Speicherplatz eingeschrieben.

Der Grund dafür ist darin zu sehen, daß der Querschnitt einer Lichtleitfaser so groß ist, daß ihm mehrere Abtastwerte zugeordnet sind. Diese Abtastwerte folgen nicht in jedem Fall unmittelbar aufeinander; denn wenn der über einen Lichtleitfaser übertragene Lichtfleck in der Wandleranordnung in mehreren Zeilen abgetastet wird, treten die einer Lichtleitfaser zugeordneten Abtastwerte zu unterschiedlichen Zeiten auf. Durch den Aufruf des Inhaltes des einer bestimmten Lichtleitfaser zugeordneten Speicherplatzes und durch Addition des der gleichen Lichtleitfaser zugeordneten Abtastwertes wird erreicht, daß am Ende jeder Speicherplatz die Summe der Abtastwerte enthält, die jeweils einer bestimmten Lichtleitfaser zugeordnet sind. - Grundsätzlich könnte man sich damit begnügen, jeder Lichtleitfaser nur einen einzigen Abtastwert zuzuordnen. Dieser eine Abtastwert könnte jedoch durch Rauscheffekte verfälscht werden, weshalb es im Hinblick auf eine Verbesserung des Signal/Rausch-Verhältnisses günstiger ist, mehrere Abtastwerte zu verwenden. Es ist daher am günstigsten, eine Zahl von n Abtastwerten zu verwenden, wobei n eine Zahl ist, die kleiner oder gleich ist der minimalen Zahl der einer Lichtleitfaser zugeordneten Abtastwerte. Für jede Lichtleitfaser ist somit in einem der Speicherplätze des Speichers 28 die Summe von n Abtastwerten gespeichert, die dem Mittelwert dieser Abtastwerte entspricht - und zwar unter der Adresse $a_1$ ein Mittelwert, der dem Punkt $x_1$ im zeilenförmigen Röntgenschattenbild zugeordnet ist, unter der Adresse $a_2$ ein der Stelle $x_2$ zugeordneter Wert usw. Infolgedessen stellt die im Speicher 28 nach aufsteigenden Adressen angeordnete Folge von Abtastwerten - bzw. der Summe u dieser Abtastwerte - ein getreues Abbild des Verlaufes der Intensität r als Funktion des Ortes x dar (vgl. Fig. 2a und 2c).

Aus dem vorangehenden wird deutlich, daß es darauf ankommt, daß in dem programmierbaren Festwertspeicher 27 für jeden Zählerstand, d.h. für jeden Abtastzeitpunkt, die Adresse (z.B. $a_1$) gespei-chert ist, die durch ihre Stellung innerhalb der Adressenfolge des Speichers 28 der räumlichen Lage ($x_1$) des Punktes entspricht, dem der Abtast-zeitpunkt ($t_1$) zugeordnet ist, zu dem der Abtastwert gewonnen wird, der im Speicher 28 unter der genannten Adresse - durch Addition - gespeichert wird. Wie dies erreicht wird, wird nachstehend anhand von Fig. 4 näher erläutert.

Danach ist vor die Lichtleiteranordnung 7 eine Blende 40 mit einer Öffnung 41 geschoben, die so bemessen ist, daß durch diese Öffnung hindurch jeweils nur eine einzige Lichtleitfaser mit konstanter Intensität bestrahlt werden kann. Die Blende 40 wird von einem Schrittmotor 42 in Zeilenrichtung verschoben, wobei die Anordnung so getroffen ist, daß durch einen Impuls die Blende jeweils um den Abstand zweier benachbarter Lichtleitfasern verschoben wird.

Die unvermeidbaren Toleranzen im Abstand zwischen den einzelnen Lichtleitfasern (auch durch Toleranzen im Faserdurchmesser bedingt) lassen sich durch Einsatz eines Mikrorechners ausgleichen, der das Intensitätsmaximum in der Nähe der erwarteten Fasermitte sucht und den Schrittmotor 42 automatisch zur tatsächlichen Fasermitte führt. Findet der Rechner keine Faser vor, liefert er einen Hinweis auf einen möglichen Faserbruch.

Die Impulse, die dem Schrittmotor von einer Stufe 43 zugeführt werden, werden gleichzeitig dem Zähleingang eines Zählers 44 zugeführt, so daß der Zählerstand der Position der Öffnung 41 bzw. der Lage x der jeweils von Strahlung getroffenen Lichtleitfaser entspricht. Die Adresse des Speicherplatz es, in den der Stand des Zählers 44 eingeschrieben werden soll, wird von dem Zähler 26 geliefert, der von dem Taktgenerator 25 synchron zur Wandleranordnung 12 gesteuert wird, so daß der Zählerstand einem bestimmten Zeitpunkt t in bezug auf den Beginn des Umsetzvorganges durch die Wandleranordnung entspricht.

Ob der Inhalt des Zählers 44 unter der durch den Speicher 26 bestimmten Adresse in den Speicher 27 programmiert wird, hängt von dem Signal am Programmiereingang 270 des Speichers ab, das von einer Vergleichsstufe 45 geliefert wird. Die Vergleichsstufe vergleicht den von der Wandleranordnung 12 gelieferten Wert mit einem Schwellenwert und bewirkt jedesmal dann das Programmieren des Speichers 27, d.h. das Einschreiben des Inhaltes des Zählers 44 unter der durch den Zähler 26 bestimmten Adresse, wenn die Amplitude des von der Wandleranordnung gelieferten Signals größer ist als der Schwellenwert. Dadurch wird folgendes erreicht.

Solange das Ausgangssignal der Wandleranordnung 12 von Wandlerelementen geliefert wird, die nicht in optischem Kontakt mit dem jeweils bestrahlten Lichtleiter stehen, ist das Ausgangssignal der Wandleranordnung praktisch gleich Null und kleiner als der Schwellenwert, so daß kein Einschreibvorgang bei dem programmierbaren Festwertspeicher 27 durchgeführt wird. Wird hingegen zu einem bestimmten Augenblick das Ausgangssignal der Wandleranordnung 12 durch ein Wandlerelement bestimmt, das vollständig von der jeweils im Strahlengang angeordneten Lichtleitfaser bestrahlt wird, erreicht das Ausgangssignal einen Wert, der oberhalb des Schwellwertes liegt, so daß in diesem Fall der jeweilige Wert x (z.B. $x_1$) unter der jeweiligen Adresse t (z.B. $t_1$) eingeschrieben wird. - Es gibt auch noch Wandlerelemente, die nur zu einem gewissen Teil durch die jeweils bestrahlte Lichtleitfaser beleuchtet werden, so daß das Ausgangssignal, das diesen Wandlern zugeordnet ist, eine geringere Amplitude hat. Ein Einschreibvorgang wird hierdurch nicht ausgelöst, weil der Schwellenwert so groß gewählt ist, daß er nur durch das Signal von Wandlerelementen überschritten werden kann, die vollständig beleuchtet werden.

Wie bereits erwähnt, sind jeder Lichtleitfaser, d.h. jedem Zählerstand x n Abtastwerte zugeordnet, d.h. n Zeitpunkte, zu denen das Ausgangssignal der Wandleranordnung 12 den Schwellenwert überschreitet. Nachdem also der jeweilige Stand des Zählers 44 unter der durch den Zähler 26 bestimmten Adresse eingeschrieben worden ist, wird der Zählerstand des Zählers 44 unverändert beibehalten, während der Stand des Zählers 26 durch den Taktgenerator 25 fortlaufend erhöht wird, bis zu einem weiteren Zeitpunkt bzw. bei einem Stand t' des Zählers 26 der Momentanwert des Ausgangssignals der Wandleranordnung 12 erneut den Schwellenwert der Vergleichsschaltung 45 überschreitet. In diesem Fall wird der Wert x erneut in den programmierbaren Festwertspeicher 27 eingeschrieben, jedoch unter der neuen Adresse t'.

Die Stufe 43, die den Schrittmotor 42 und den Zähler 44 steuert, ist als Untersetzerschaltung im Verhältnis 1:n, d.h. als 1:n Frequenzteiler, ausgebildet. Nach jeweils n Impulsen erzeugt dieser Frequenzteiler einen Ausgangsimpuls, so daß die Blende 40 zur nächsten Lichtleitfaser verschoben wird und der Stand des Zählers 44 um 1 erhöht wird. Gleichzeitig wird über den Ausgang der Stufe 43 der Zähler 26 auf Null gesetzt, und es werden die (ersten) n Adressen bestimmt, denen der neue Stand des Zählers 44 zugeführt wird. Das ganze wiederholt sich so lange, bis alle Lichtleitfasern einmal von Strahlung getroffen worden sind.

Anstatt die Blende vor den Lichtleitern zu verschieben, ist es auch möglich, den Leuchtpunkt einer Katodenstrahlröhre auf jeweils eine Lichtleitfaser abzubilden und diesen Leuchtpunkt so zu verschieben, daß er nacheinander auf jeweils einer Lichtleitfaser abgebildet wird. Anstatt einer mechanischen Verschiebung der Blende wäre in diesem Fall also eine elektronische Verschiebung des Leuchtpunktes durch das Taktsignal erforderlich. - Es kann auch ein programmierbarer Festwertspeicher benutzt werden, der keinen besonderen Programmiereingang besitzt und bei dem das Einschreiben dadurch erfolgt, daß die Versorgungsspannung erhöht wird. In diesem Fall ist die Versorgungsspannung immer dann zu erhöhen, wenn das Ausgangssignal des Speichers 12 den Schwellenwert der Vergleichsschaltung 45 überschreitet.

Es ist ersichtlich, daß die Schaltung nach Fig. 3 - und ggf. auch die nach Fig. 4 - durch einen geeignet programmierten Microcomputer ersetzt werden kann. In diesem Fall würde ebenso wie bei der Schaltung nach Fig. 3 das Sortieren vor dem Einschreiben in den Speicher 28 erfolgen. In Fig. 5 ist ein mit Hilfe eines Microcomputers realisiertes Prinzipschaltbild einer Sortierschaltung dargestellt, bei der der Sortiervorgang beim Auslesen aus dem Speicher erfolgt.

Die Abtastwerte aus dem Analog-Digital-Wandler werden dabei direkt einem Speicher 28 zugeführt, so daß die Folge der Abtastwerte in dem Speicher 28 der Signalfolge gemäß Fig. 2b entspricht. Es ist ein weiterer Speicher 51 vorgesehen, dessen Speicherplätzen ein oder mehrere Speicherplätze im Speicher 28 zugeordnet werden. Zu diesem Zweck ist ein Microcomputer 50 vorgesehen, der die im Speicher 28 gespeicherten Abtastwerte der Reihe nach aufruft und sie in den Speicher 51 jeweils unter einer bestimmten Adresse einschreibt - ggf. nach Addition zu dem in diesem Speicher schon enthaltenen Wert, wie in Verbindung mit Fig. 3 erläutert. Der Microcomputer enthält einen nicht näher dargestellten Festwertspeicher, in dem die Adressenzuordnungen zwischen dem Speicher 28 und dem Speicher 51 gespeichert sind und der in gleicher Weise - vorzugsweise unter Benutzung des Microcomputers - programmiert werden kann, wie anhand von Fig. 4 erläutert. Am Ende des Sortiervorganges entspricht die Folge der nach einer bestimmten Adressenfolge gespeicherten Abtastwerte daher wiederum derjenigen nach Fig. 2c, so daß der ursprüngliche Intensitätsverlauf durch Auslesen des Speichers 51 mit dieser Adressenfolge rekonstruiert werden kann.

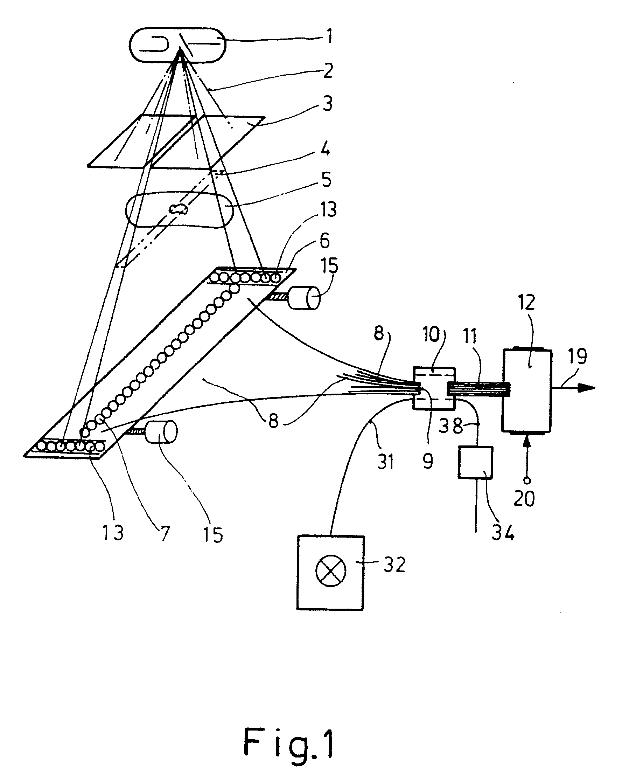

Wie aus Fig. 1 ersichtlich, enthält das Lichtleiterbündel 8 außer den zur Abbildung des Röntgenschattenbildes dienenden Lichtleitfasern 7 beiderseits des zeilenförmigen, vom Röntgenstrahlenbündel ausgeleuchteten Bereichs weitere Lichtleitfasern, die der Regelung der Position der Szintillatoranordnung 6 bzw. des Lichtleitfaserbündels 8 dienen. Das von diesen Lichtleitern 13 erzeugte Licht wird dem Bildverstärker 10 zugeführt, dessen Ausgangsbild von der Wandleranordnung 19 in ein elektrisches Signal umgesetzt wird.

Das über die Lichtleiter 13 übertragene Licht bzw. die ihm im Speicher 28 zugeordneten Abtastwerte werden von dem Microcomputer 50 einem weiteren Speicher 52 - mit geringerer Speicherkapazität - zugeführt und stehen an dessen Ausgang als (digitale) Regelsignale zur Verfügung, wodurch zwei Stellmotoren 15 (Fig. 1) so beaufschlagt werden können, daß sie die Zeile der Lichtleiter 7 immer parallel zum Schlitz der Blendenanordnung 3 ausrichten.

Ein weiteres Steuersignal kann dem Eingang des Bildverstärkers 10 von einer Lichtquelle 32 mit außerordentlich konstanter Intensität (sogenannte Beta-Lichtquelle) über eine Lichtleitfaser 31 zugeführt werden, so daß die Helligkeit an dem dem Ausgang dieser Lichtleitfaser zugeordneten Punkt am Ausgang des Bildverstärkers 10 ein Maß für die Verstärkung des Bildverstärkers 10 ist.

Dieses Licht am Ausgang des Bildverstärkers 10 kann durch eine Lichtleitfaser 38 erfaßt, von einem gesonderten Wandler 34 in ein elektrisches Signal umgesetzt und zur Steuerung der Verstärkung des Bildverstärkers 10 herangezogen werden. Es könnte aber auch dem Steuereingang 21 des in seiner Verstärkung steuerbaren Verstärkers 22 zugeführt werden, so daß die Gesamtverstärkung vom Eingang des Bildverstärkers bis zum Eingang des Analog-Digital-Wandlers 23 konstant bleibt.

## Patentansprüche

1. Röntgengerät mit einem Röntgenstrahler (1) und einer Blendenanordnung (3) zum Erzeugen eines fächerförmigen einen Untersuchungsbereich (5) durchsetzenden Strahlenbündels (4), mit einer flächigen Szintillatoranordnung (6) zum Umsetzen des durch das Strahlenbündel vom Untersuchungsbereich erzeugten zeilenförmigen Röntgenschattenbildes in ein längerwelliges Strahlungsbild, einer mit der Szintillatoranordnung gekoppelten Lichtleiteranordnung (8), einer Wandleranordnung (12), die das längerwellige Strahlungsbild sequentiell in elektrische Signale umsetzt, und mit einem Speicher (28) zum Speichern von Abtartwerten der elektrischen Signale, dadurch gekennzeichnet, daß die Szintillatoranordnung (6) über die Lichtleiteranordnung (8) mit einem Bildverstärker (10) gekoppelt ist, dessen Ausgangsbild auf die Wandleranordnung (12) abbildbar ist, und daß eine Sortierschaltung (24..27; 50) 50 vorgesehen ist, daß sie die Abtastwerte des elektrischen Signals (S) beim Einschreiben in bzw. beim Auslesen aus dem Speicher so vertauscht, daß ihre Folge der Folge der Helligkeitswerte (r) in dem zeilenförmigen Röntgenschattenbild entspricht.

2. Röntgengerät nach Anspruch 1, dadurch gekennzeichnet, daß ein erster Speicher (28) vorgesehen ist, in dessen Speicherplätze die vom Wandler (12) stammenden Abtastwerte einschreibbar sind, daß die Sortierschaltung (50) so ausgelegt ist, daß sie Abtastwerte aus dem ersten Speicher ausliest und in einen zweiten Speicher (51) einschreibt, wobei sie nach einer vorgegebenen Tabelle jeder Adresse im ersten Speicher eine Adresse im zweiten Speicher zuordnet, derart, daß beim Auslesen des zweiten Speichers (51) in einer vorgegebenen Adressenfolge die Folge der Abtastwerte dem Röntgenschattenbild entspricht (Fig. 5).

3. Röntgengerät nach Anspruch 1, dadurch gekennzeichnet, daß die Sortierschaltung (24..27) so ausgelegt ist, daß sie beim Einlesen in den Speicher (28) die Abtastwerte unter einer durch eine vorgegebene Tabelle gegebenen Adresse speichert, derart, daß beim Auslesen des Speichers mit einer vorgegebenen Adressenfolge die Abtastwerte in einer dem Röntgenschattenbild entsprechenden Folge erscheinen (Fig. 3).

4. Röntgengerät nach Anspruch 1, dadurch gekennzeichnet, daß Lichtleiter (13) 50 vorgesehen sind, daß sie die Helligkeit der Szintillatoranordnung außerhalb des zeilenförmigen Röntgenschattenbildes erfassen und dem Eingang des Bildverstärkers (10) zuführen.

## Claims

1. An X-ray apparatus comprising an X-ray source (1) and diaphragm means (3) for the formation of a fan-shaped radiation beam (4) which passes through an examination zone (5), a planar scintillator array (6) for converting the line-shaped X-ray shadow image of the examination zone, formed by the radiation beam, into a longer wavelength radiation image, a light-conductor array (8) which is coupled to the scintillator array, a transducer array (12) which sequentially converts the longer wavelength radiation image into electric signals, and a memory (28) for the storage of scanning values of the electric signals, characterized in that the scintillator array (6) is coupled to an image intensifier (10) via the light-conductor array (8), it being possible to image the intensifier output image on the transducer array (12), and in that there is provided a sorting circuit (24 ... 27; 50) so that, during writing into or reading from the memory, the scanning values of the electric signal (S) are rearranged in such a way that their sequence corresponds to the sequence of the brightness values (r) in the line-shaped X-ray shadow image.

2. An X-ray apparatus as claimed in Claim 1, characterized in that there is provided a first memory (28) in the storage locations of which the scanning values originating from the transducers (12) can be written, the sorting circuit (50) being constructed so that it reads the scanning values from the first memory and writes these values into a second memory (51), assigning an address in the second memory to each address in the first memory in conformity with a predetermined table in such a way that upon reading of the second memory (51) in a predetermined address sequence the sequence of the scanning values corresponds to the X-ray shadow image (Fig. 5).

3. An X-ray apparatus as claimed in Claim 1, characterized in that the sorting circuit (24 ... 27) is constructed so that upon writing into the memory (28) it stores the scanning values at an address dictated by a predetermined table in such a way that upon reading of the memory in a redetermined address sequence the scanning values appear in a sequence corresponding to the X-ray shadow image (Fig. 3).

4. An X-ray apparatus as claimed in Claim 1, characterized in that there are provided light conductors (13) such that they detect the brightness of the scintillator array outside the line-shaped X-ray shadow image for transmission to the input of the image intensifier (10).

**Revendications**

1. Appareil à rayons X comportant une source de rayons X (1) et un dispositif à diaphragmes (3) pour la production d'un faisceau de rayons en éventail (4) traversant une zone d'examen, un dispositif scintillateur plat (6) pour la conversion du skiagramme en forme de ligne produit à partir de la zone d'examen par le faisceau de rayons en une image de rayonnement de plus grande longueur d'onde, un dispositif de guidage de lumière (8) couplé au dispositif scintillateur, un dispositif transducteur (12) qui convertit l'image de rayonnement de plus grande longueur d'onde séquentiellement en signaux électriques et une mémoire (28) pour le stockage de valeurs d'échantillonnage des signaux électriques, caractérisé en ce que le dispositif scintillateur (6) est couplé, par l'intermédiaire du dispositif de guidage de lumière (8), à un amplificateur d'image (10) dont l'image de sortie peut être formée sur le dispositif transducteur (12) et qu'un circuit de triage (24, .., 27; 50) est prévu de manière telle qu'il permute les valeurs d'échantillonnage du signal électrique (s), lors de l'inscription dans la mémoire et lors de la lecture de cette mémoire, pour que leur ordre de succession corresponde à celui des valeurs de luminosité (r) dans le skiagramme en forme de ligne.

2. Appareil à rayons X suivant la revendication 1, caractérisé en ce qu'une première mémoire (28) est prévue, dans les emplacements de stockage de laquelle les valeurs d'échantillonnage provenant du transducteur (12) peuvent être inscrites, que le circuit de triage (50) est conçu tel qu'il extrait les valeurs d'échantillonnage par lecture de la première mémoire et les inscrit dans une seconde mémoire (51), en associant, d'après une table prédéterminée, à chaque adresse dans la première mémoire, une adresse dans la seconde mémoire, pour que, lors de la lecture de la seconde mémoire (51), selon un ordre de succession d'adresses prédéfini, la succession des valeurs d'échantillonnage corresponde au skiagramme (Fig. 5).

3. Appareil à rayons X suivant la revendication 1, caractérisé en ce que le circuit de triage (24, .., 27) est conçu tel que, lors de l'introduction dans la mémoire (28), il stocke les valeurs d'échantillonnage sous une adresse donnée par une table prédéterminée, pour que, lors de l'extraction par lecture de la mémoire, selon un ordre de succession d'adresses prédéterminé, les valeurs d'échantillonnage apparaissent dans un ordre de succession correspondant au skiagramme (Fig. 3).

4. Appareil à rayons X suivant la revendications 1, caractérisé en ce que des conducteurs de lumière (13) sont prévus tels qu'ils détectent la luminosité du dispositif scintillateur à l'extérieur du skiagramme en forme de ligne et l'appliquent à l'entrée de l'amplificateur d'image (10).

Fig.1

Fig.2

a)

b)

c)

Fig.5

Fig.3

Fig.4